## Europäisches Patentamt

## European Patent Office

## Office européen des brevets

(11) Publication number: **0 085 452**
**B1**

(12) **EUROPEAN PATENT SPECIFICATION**

(45) Date of publication of patent specification: **04.11.87**

(51) Int. Cl.⁴: **A 63 B 9/00** // F16B7/18, E04G7/18

(21) Application number: **83200076.4**

(22) Date of filing: **18.01.83**

(54) **Connecting device for round poles of a play implement.**

(30) Priority: **20.01.82 NL 8200204**

(43) Date of publication of application: **10.08.83 Bulletin 83/32**

(45) Publication of the grant of the patent: **04.11.87 Bulletin 87/45**

(84) Designated Contracting States: **AT BE CH DE FR GB IT LI LU NL SE**

(56) References cited:
FR-A- 612 018
FR-A-1 079 838
GB-A- 897 382
US-A-3 046 040
US-A-3 814 416
US-A-4 252 313

(73) Proprietor: **Speelhout B.V.**
**Schimminck 13**
**NL-5300 AB Zaltbommel (NL)**

(72) Inventor: **Te Duits, Everhardus Hendrikus**
**Arminiushof 17**
**NL-1216 KC Hilversum (NL)**

(74) Representative: **Urbanus, Henricus Maria, Ir. et al**
**c/o Vereenigde Octrooibureaux Nieuwe Parklaan 107**
**NL-2587 BP 's-Gravenhage (NL)**

Courier Press, Leamington Spa, England.

# Description

The invention relates to a device for connecting at least two round poles of a play implement comprising clamping means extending through bores in the poles and bearing via supporting elements on outer faces on opposite sides of the poles, said clamping means comprising a bolt and a nut element and a spacing element extending between the poles coaxially with the bolt of said clamping means and having curved contact faces intimately engaging the poles at least along an outer edge of said curved contact faces. A device of this type is known from DE—U—8,017,526.

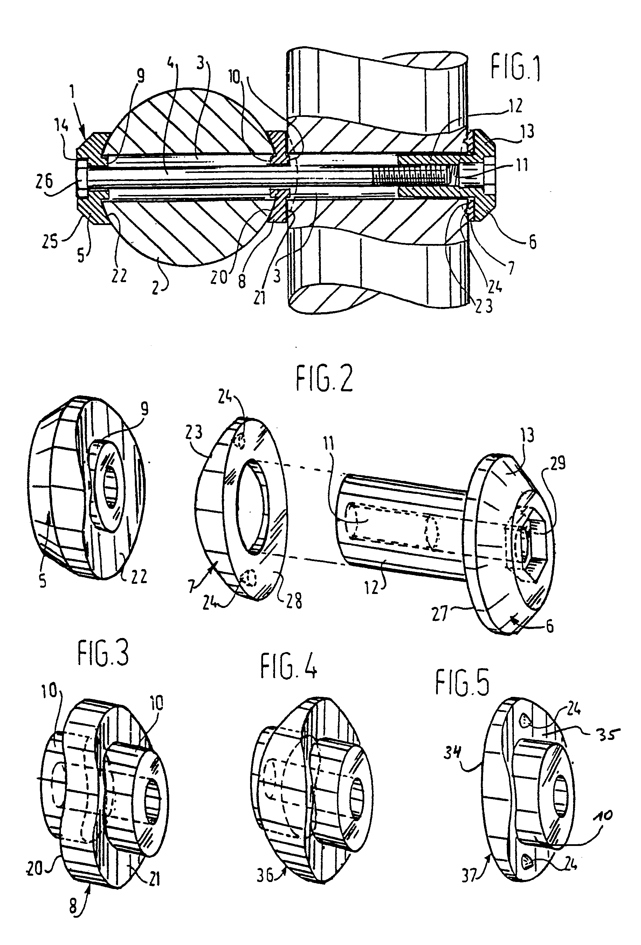

In the prior arrangement, the spacing element between the poles serves to prevent a playing child from jamming its fingers in the gap between the two poles. In play elements built up from poles, there is in addition commonly the danger that children get wounded from the bolt head and/or the nut. In the prior arrangement, according to Fig. 2, this appears to be prevented by these parts being either smoothed down, or counter-sunk in suitable holes formed in the poles in contiguity with the bore. In such a construction, however, the bolt is subject to rather substantial shear forces exercised when children climb upon the play implement and thus forces are exercised on the poles tending to move these relatively to each other. In the prior arrangement, there is further indicated how a bolt head projecting from a part can be rounded, but this only to secure a round pole to a flat plate. There is no showing as to how an injury-free connection can be formed in the case of two round poles.

It is an object of the present invention to provide a device for connecting at least two round poles of a clay implement, by means of which not only is the risk of injury suppressed to a substantial extent, but furthermore shear forces are prevented from being exercised on the bolt elements.

The above object is achieved with a device of the kind described in the opening paragraph hereof, wherein between the head of said bolt and the adjacent pole there is provided a supporting element having a recess for receiving the head of said bolt and a curved contact face intimately engaging said pole, at least along an outer edge of said curved contact face, and having a coaxial shoulder protruding from said curved face, said shoulder extending into a substantially matching bore in said pole, and that said nut element is provided with an internally screwthreaded shank, said shank extending into a substantially matching bore of the other pole, said nut element further comprising a head protruding beyond the shank and having an annular flat face directed towards the shank, which engages a ring having a curved contact face on the other side, and that said spacing element is provided with two coaxial shoulders protruding on opposite sides from said curved contact faces and each extending into a substantially matching bore in the adjacent pole.

In a suitable embodiment according to the invention, one or more of said curved contact faces is provided with lugs protruding from said curved contact face.

In case the poles have to be interconnected at an arbitrary angle, the spacing element of the device of the invention comprises two rings engaging one another by flat side faces and having a curved contact face on the side opposite the flat face, from which curved contact face said shoulder of said ring protrudes. The rings can relatively turn along the flat faces, so that any angle can be formed.

In a further development of the invention relatively co-operating arresting members are provided in each flat side face of the partial rings. In this way it is yet possible to obtain the advantage of guarding the poles against a relative turn. The arresting members may have the shape of sharp tips penetrating into the material of the opposite partial ring or of teeth arranged at fixed angular distances, for example, angles of 15°. In the latter case the spacing element formed by the partial rings permits of establishing an angular connection in which the angle formed is a multiple of 15°.

For interconnecting poles at a larger distance the spacing element of the device according to the invention is formed by a pipe or bar provided near each end with a ring, each ring being shaped so as to act as one of said curved contact faces for intimately engaging a pole. Such a spacing element may be advantageously employed as a rung of a ladder or as a horizontal bar. The matching contact faces ensure the desired safety, whilst at the same time a very firm connection is established which prevents the poles from turning relatively to one another.

In the case of such a long spacing element the pipe or rod preferably has at each end a hole with internal screw-thread. Then the clamping elements are two bolts passed each through one pole and engaging the tapped hole of the spacing element.

According to the invention a coaxial shoulder is provided on the side(s) of a curved contact face of the different elements of the device. These coaxial shoulders each penetrate into a matching bore in the pole concerned and thus effectively absorbs longitudinal forces or shear forces exerted on the element.

The invention will be described more fully with reference to the accompanying drawings.

Fig. 1 is a longitudinal sectional view of a device embodying the invention.

Fig. 2 is a perspective view of part of the device of Fig. 1.

Figs. 3 and 5 show three embodiments of a spacing element.

Fig. 6 shows a still further embodiment.

Fig. 7 shows a spacing element for connecting poles at a larger distance.

Fig. 8 shows a further embodiment of a spacing element.

Fig. 9 is a perspective view of a play implement formed by poles connected by devices embodying the invention.

By means of the device embodying the invention two round poles 2 of a play implement are connected with one another. For this purpose bores 3 are made in the poles 2. Through the bores 3 extends a bolt 4 serving as a clamping means of the device 1. The head 26 of the bolt 4 bears on a supporting element 5, which is in contact with the left-hand pole 2. The screwthreaded end of the bolt 4 engages a nut element 6 on the other side of the device. The nut element 6 has a shank 12 with a tapped hole 11 and a head 13. On its side facing the shank 12 the head 13 has a flat face. The flat face of the head 13 bears on a flat face of a ring 7. The ring 7 bears on the pole 2. According to the invention a spacing member 8 is arranged between the poles 2 and the connecting device. This spacing member 8 holds the poles 2 at a minimum distance so that no hazardous, narrow, wedge-shaped gap is formed between the poles. The spacing element has curved faces 20, 21 which intimately engage the poles at least along an outer edge of said curved faces.

According to a further aspect of the invention the supporting element 5 and the ring 7 associated with the nut element 6 also have curved contact faces 22 and 23 respectively, which fit to the surface of the poles 2. According to an aspect of the invention the supporting element 5 and the spacing element 8 are provided with shoulders 9 and 10 respectively. Any shear forces occurring between the poles 2 are transferred by the shoulders 10 of the spacing element 8. In this way the connecting device itself is prevented from being exposed to shear forces. By the shoulder 9 the supporting element 5 is correctly positioned with respect to the bore 3. Fig. 1 furthermore shows that the supporting element 5 has a recess 14 for receiving the head 26 of the clamping element or else the bolt 4. The outer edge of the supporting element 5 has a bevelled face 25 so that the risk of injury due to sharp edges of the supporting element 5 itself or the bolt head 26 is eliminated. The supporting element 5 and nut element 6 with the ring 7 are shown in detail in Fig. 2. The spacing element 8 is shown in detail in Fig. 3.

Fig. 2 shows in detail the above-described elements of the connecting device embodying the invention shown in Fig. 1. The supporting element 5 and the ring 7 are, by their respective curved contact faces 23 and 22, in engagement with the poles 2. The nut element 6 can be turned because the underside 27 of the head 6 is flat and is in contact with the flat face 28 of the ring 7. For mounting the device the bolt 4 (not shown in Fig. 2) is passed through the supporting element 5 and the poles and at the other end the nut element 6 passed through the ring 7 is screwed onto the bolt. For tightening the connection the head 13 of the nut element 6 has a hexagonal recess 29 into which a hexagonal wrench can be inserted.

Figs. 1 and 2 furthermore show that the ring 7 is provided with lugs 24 which penetrate into the material of the pole 2 and have the effect of guard elements. Apart from the curved face of the ring 7, the curved face 22 of the supporting element 5 or the curved faces of the spacing elements may also be provided with lugs.

The centre lines of the curvatures of the curved contact faces 20 and 21 on the two sides of the spacing element 8, as shown in Fig. 3, are at right angles to one another so that the poles are also .connected at an angle of 90° with one another. The spacing element 8 furthermore is provided with shoulders 10 on both sides.

The spacing element 36 shown in Fig. 4 has curved contact faces on both sides, the centre lines of the curvatures extending parallel to one another. The spacing element 36 is used for interconnecting poles in parallel position. It is also provided with shoulders.

The spacing element as shown in Fig. 5 has on one side a flat face 34 and on the other side a curved contact face 35. By using as spacing elements two partial rings 37 engaging one another by their flat faces 34 poles can be interconnected at any arbitrary angle because the curved faces 35 of the respective partial rings can be relatively turned at any angle. The spacing element 37 is also provided with shoulders.

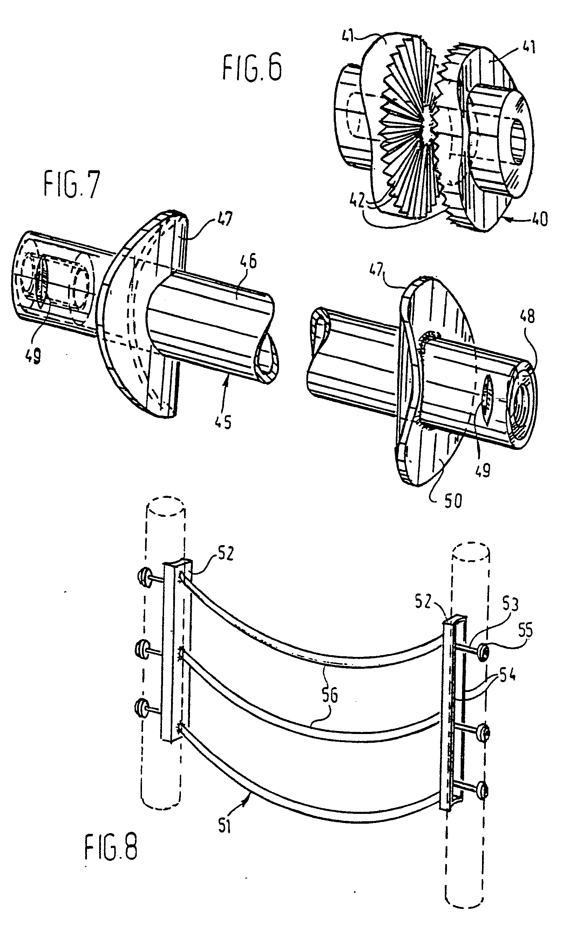

The spacing element 40 of Fig. 6 comprises two partial rings 41. The partial rings 41 basically correspond with the partial ring 37 of Fig. 5, but arresting members formed by a toothing 42 are provided in the flat face. The teeth may be disposed at relative angular distances of 15°. In this way the two partial rings 41 can be employed for a connecting device embodying the invention to connect poles with one another at an angle of an integral multiple of 15°. The arresting members provide the advantage that the poles are guarded against a turn in the selected angular position. Instead of using cooperating teeth, sharp members may be used in one or in both flat faces to penetrate into the material of the flat face of the opposite partial ring 41.

The spacing element 45 of Fig. 7 comprises a pipe 46 provided near the ends with rings 47 welded thereto. The rings 47 are chosen so that the curved contact face 50 is formed on the side facing the associated end of the pipe 46. Into the ends of the pipe 46 are introduced screwthreaded sleeves 48, which are guarded in place by means of a depression 49. The spacing element 45 can be connected at both ends with a pole by means of a bolt bearing on the supporting element, for example, the supporting element 5 of Fig. 2. The spacing element 45, as is shown in Fig. 7, serves as the rung of a ladder, as a horizontal bar or as a part of railing. Although the distance between the poles to be interconnected, when using a spacing element 45, is relatively large, the additional advantage of the invention of guarding the poles against a relative turn is nevertheless obtained.

Fig. 8 shows a further development of the spacing element shown in Fig. 7. This spacing element 51 comprises three bars 56 secured at both ends to locking members 52. The locking members 52 are constructed so that their outer rim 54 forms the curved contact face. To the locking members 52 are fastened bolts 53 which can be passed through bores in the pole concerned, whilst a nut 55 can be screwed onto the other end.

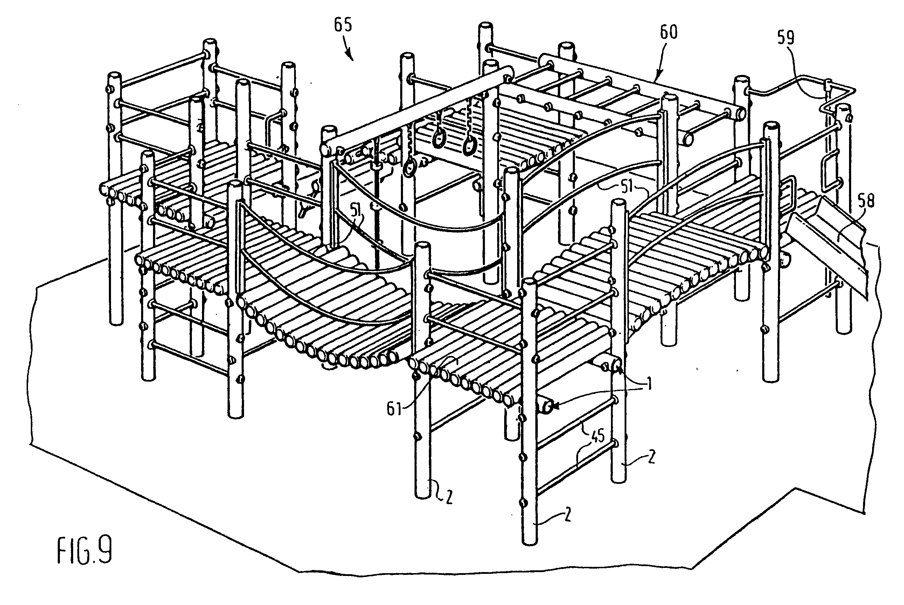

As is shown in Fig. 9, the spacing element 51 may be used with the curvature of the bars 56 pointing upwards or downwards. The play implement 65 comprises a large number of poles 2, which are interconnected in different ways by means of the device embodying the invention. In the play implement 65 are formed foot boards 61 and, in addition, a slide 58 and a suspension bridge 60. There is also provided a slide rod 59. It will be obvious that by means of the connecting device embodying the invention a large number of different play appliances can be formed. Fig. 9 clearly shows the various above-mentioned embodiments of the connecting device in accordance with the invention. When the device embodying the invention is employed, children, even when playing boisterously, will not get jammed or injured, whilst by the additional advantage of the invention in guarding the interconnected poles against a relative turn a very rigid and robust construction is obtained.

**Claims**

1. A device for connecting at least two round poles (2) of a play implement comprising clamping means extending through bores (3) in the poles and bearing via supporting elements on outer faces on opposite sides of the poles, said clamping means comprising a bolt (4) and a nut element (6), and a spacing element (8) extending between the poles coaxially with the bolt (4) of said clamping means and having curved contact faces (20, 21) intimately engaging the poles (2) at least along an outer edge of said curved contact faces, characterized in that between the head (26) of said bolt (4) and the adjacent pole there is provided a supporting element (5) having a recess (14) for receiving the head (26) of said bolt (4) and a curved contact face (22) intimately engaging said pole, at least along an outer edge of said curved contact face (22), and having a coaxial shoulder (9) protruding from said curved face (22), said shoulder (9) extending into a substantially matching bore in said pole, and that said nut element (6) is provided with an internally screwthreaded shank (12), said shank (12) extending into a substantially matching bore of the other pole, said nut element (6) further comprising a head (13) protruding beyond the shank (12) and having an annular flat face (27) directed towards the shank, which engages a ring (7) having a curved contact face (23) on the other side, and that said spacing element (8) is provided with two coaxial shoulders (10) protruding on opposite sides from said curved contact faces (20, 21) and each extending into a substantially matching bore in the adjacent pole.

2. A device as claimed in claim 1, characterized in that one or more of said curved contact faces (20, 21, 22, 23) is provided with lugs (24) protruding from said curved contact face.

3. A device as claimed in claim 1 or 2, characterized in that said spacing element (8) comprises two rings (37) engaging one another by flat side faces (34) and having a curved contact face (35) on the side opposite the flat face (34), from which curved contact face (35) said shoulder (10) of said ring (37) protrudes.

4. A device as claimed in claim 1 or 2, characterized in that said spacing element (40) comprises two rings (41) engaging one another by side faces formed with co-operating arresting members (42).

5. A device as claimed in claim 1 or 2, characterized in that said spacing element (45) is a pipe (46) or bar provided near each end with a ring (47), each ring being shaped so as to act as one of said curved contact faces for intimately engaging a pole.

**Patentansprüche**

1. Vorrichtung zum Verbinden von mindestens zwei runden Pfosten (2) eines Spielgeräts, mit Klemmitteln, die sich durch Bohrungen (3) in den Pfosten hindurcherstrecken und sich über Unterstützungselemente an Außenflächen einander gegenüberliegender Seiten der Pfosten anliegen, wobei die Klemmittel einen Bolzen (4) und ein Mutternelement (6) aufweisen und ein Abstandselement (8) sich zwischen den Pfosten koaxial mit dem Bolzen (4) des Klemmittels erstreckt und gekrümmte Oberflächen (20, 21) aufweist, die zumindest entlang eines äußeren Rands der gekrümmten Kontaktfläche eng an den Pfosten (2) anliegen, dadurch gekennzeichnet, daß zwischen dem kopf (26) des Bolzens (4) und des unmittelbar benachbarten Pfostens ein Unterstützungselement (5) mit einer Ausnehmung (14) zur Aufnahme des Kopfs (26) des Bolzens (4) und eine gekrümmte Kontaktfläche (22), die sich entlang mindestens eines äußeren Rands der gekrümmten Kontaktfläche (22) eng an den Pfosten anlegt, vorgesehen ist und wobei es eine koaxiale Schulter (9) aufweist, die von der gekrümmten Fläche (22) absteht und die sich in eine im wesentlichen passende Bohrung in den Pfosten hineinerstreckt, und daß das Mutternelement (6) mit einem ein Innengewinde aufweisenden Ansatz (12) versehen ist, der sich in eine im wesentlichen passende Bohrung des anderen Pfostens hineinerstreckt, wobei das Mutternelement (6) des weiteren einen über den Ansatz (12) hervortretenden Kopf (13) aufweist, der eine zum Ansatz gerichtete ringförmige ebene Fläche 27 besitzt, die mit einem Ring (7) zusammenwirkt, der auf der anderen Seite eine gekrümmte Kontaktfläche (23) besitzt und daß das Abstandselement (8) mit zwei koaxialen Schultern (10) versehen ist, die aneinander abgewandten Seiten von den gekrümmten Kontaktflächen (20, 21) abstehen und sich jweils in im wesentlichen passende Bohrungen in den benachbarten Pfosten hineinerstrecken.

2. Vorrichtung nach Anspruch 1, dadurch gekennzeichnet, daß eine oder mehrere der gekrümmten Kontaktflächen (20, 21, 22, 23) mit Vorsprüngen (24) versehen ist/sind, die an der

genannten gekrümmten Kontaktfläche hervortreten.

3. Vorrichtung nach Anspruch 1 oder 2, dadurch gekennzeichnet, daß das Abstandselement (8) aus zwei Ringen (37) besteht, die mit einer ebenen Seitenfläche (34) aneinander anliegen und die eine gekrümmte Kontaktfläche (35) auf jeder der ebenen Fläche abgewandten Seite (34) versehen sind, von welcher gekrümmten Kontaktfläche (35) die Schulter (10) des Rings (37) hervorsteht.

4. Vorrichtung nach Anspruch 1 oder 2, dadurch gekennzeichnet, daß das Abstandselement (40) aus zwei Ringen (41) besteht, die miteinander über Seitenflächen in Eingriff stehen, die mit ineinander greifenden Arretierungsmittel (42) ausgebildet sind.

5. Vorrichtung nach Anspruch 1 oder 2, dadurch gekennzeichnet, daß das Abstandselement (45) ein Rohr (46) oder ein Balken ist, der nahe eines jeden Endes mit einem Ring (47) versehen ist, wobei jeder Ring so geformt ist, daß er als eine der zur engen Anlage an einen Pfosten vorgesehenen gekrümmten Kontaktfläche dient.

**Revendications**

1. Un dispositif de liaison entre au moins deux poteaux ou poutres cylindriques (2) d'un jeu, comprenant des moyens de serrage passant par des trous (3) et agissant grâce à des éléments de support sur les faces des côtés opposés des poteaux ou poutres, lesdits moyens de serrage comprenant une vis (4) et un élément-écrou (6), un élément d'écartement (8) étant disposé entre les poteaux ou poutres coaxialement avec la vis (4) desdits moyens de serrage et présentant des surfaces de contact incurvées (20, 21) coopérant étroitement avec les poteaux ou poutres (2) au moins le long d'un bord extérieur desdites surfaces de contact, caractérisé en ce que, entre la tête (26) de ladite vis (4) et le poutre ou le poteau adjacent, est prévu un élément de support (5) présentant un évidement (14) pour recevoir la tête (26) de ladite vis (4) et une surface de contact (22) incurvée coopérant étroitement avec ledit poteau ou poutre, au moins le long d'un bord extérieur de ladite surface de contact (22), et présentant un épaulement coaxial (9) saillant de ladite surface incurvée (22), ledit épaulement (9) pénétrant dans un trou sensiblement correspondant audit poteau ou poutre, ledit élément écran (6) étant pourvu d'un tronc fileté intérieurement (12) pénétrant dans un trou sensiblement correspondant de l'autre poteau ou poutre, ledit élément-écrou (6) comprenant de plus une tête (13) saillant par rapport au tronc (12) et présentant une surface annulaire plane (27) tournée vers le tronc, qui coopère avec un anneau (7) présentant de l'autre côté une surface de contact (23), ledit élément d'écartement (8) étant pourvu de deux épaulements coaxiaux (10) saillant desdites surfaces de contact incurvées (20, 21) sur les côtés opposés et pénétrant chacun dans un trou sensiblement correspondant de la poutre ou du poteau adjacent.

2. Un dispositif tel que revendiqué à la revendication 1 caractérisé en ce qu'une ou plusieurs desdites surfaces de contact (20, 21, 22, 23) sont pourvues d'ergots saillant desdites surfaces de contact.

3. Un dispositif tel que revendiqué à la revendication 1 ou 2 caractérisé en ce que ledit élément d'écartement (8) comprend deux anneaux (37) coopérant entre eux par des surfaces latérales planes et présentant chacun une surface de contact incurvée (35) du côté opposé de la surface plane (34), ledit épaulement (10) dudit anneau (37) saillant par rapport à ladite surface de contact incurvée (35).

4. Un dispositif tel que revendiqué à la revendication 1 ou 2 caractérisé en ce que l'élément (40) comprend deux anneaux (41) coopérant entre eux par des surfaces latérales présentant des organes de blocage (42) coopérants.

5. Un dispositif tel que revendiqué à la revendication 1 ou 2 caractérisé en ce que ledit élément d'écartement (45) est un tube (46) ou une barre présentant au voisinage de chaque extrémité un anneau (47), chaque anneau étant conformé de façon à travailler comme l'une desdites surfaces de contact incurvées pour coopérer étroitement avec un poteau ou poutre.

FIG.1

FIG.2

FIG.3

FIG.4

FIG.5

FIG.6

FIG.7

41

41

42

40

47

46

49

45

47

48

49

50

52

52

53

55

54

56

51

FIG.8

FIG.9